# Europäisches Patentamt

## European Patent Office

## Office européen des brevets

(19)

(11) Veröffentlichungsnummer: **0 058 365**
**B1**

(12) **EUROPÄISCHE PATENTSCHRIFT**

(45) Veröffentlichungstag der Patentschrift:
**29.08.84**

(51) Int. Cl.³: **A 61 K 35/78, B 01 D 11/02**

(21) Anmeldenummer: **82100878.6**

(22) Anmeldetag: **08.02.82**

(54) Verfahren zur Gewinnung von Inhaltsstoffen der Kamille durch Extraktion mit Kohlendioxid.

(30) Priorität: **16.02.81 DE 3105557**

(43) Veröffentlichungstag der Anmeldung:
**25.08.82 Patentblatt 82/34**

(45) Bekanntmachung des Hinweises auf die Patenterteilung:
**29.08.84 Patentblatt 84/35**

(84) Benannte Vertragsstaaten:
**AT BE CH DE FR GB IT LI LU NL SE**

(56) Entgegenhaltungen:
**DE - A - 2 709 033**

**CHEMICAL ABSTRACTS, Band 90, Nr. 14, 2. April 1979, Seite 377, Nr. 109844n, Columbus, Ohio, USA e. STAHL et al.: "Extraction of German chamomile with supercritical gases"**
**CHEMICAL ABSTRACTS, Band 86, Nr. 6, 7. Februar 1977, Seite 286, Nr. 34143q, Columbus, Ohio, USA T.K. ROSLYAKOVA et al.: "Selection of optimal conditions for conversion of prochamazulenes to chamazulene in a carbon dioxide extract from Matricaria recutita"**

(73) Patentinhaber: **Degussa Aktiengesellschaft, Weissfrauenstrasse 9, D-6000 Frankfurt am Main 1 (DE)**

(72) Erfinder: **Behr, Norbert, Kanalweg 19, D-8501 Burgthann (DE)**
Erfinder: **v. Ettingshausen, Othmar, Dr., Nettetalstrasse 5, D-4000 Düsseldorf (DE)**
Erfinder: **van der Mei, Henk, Am Hisskamp 4, D-3260 Rinteln 3 (DE)**
Erfinder: **Wüst, Reinhold, Jahnstrasse 23, D-4044 Kaarst 1 (DE)**

## Beschreibung

Die Erfindung betrifft die Gewinnung von Inhaltsstoffen der Kamille durch Extraktion mit verflüssigtem Kohlendioxid.

Die Kamille enthält zahlreiche Duftstoffe, Aromasubstanzen und heilwirksame Inhaltsstoffe, die überwiegend sehr temperaturempfindlich sind. Der Kamillentee, der im Haushalt durch Überbrühen der Droge mit heißem Wasser hergestellt wird, enthält daher nur noch einen Bruchteil der ursprünglich in der Kamille vorliegenden Inhaltsstoffe. Außerdem ist ein Teil der Inhaltsstoffe nicht genügend wasserlöslich und verbleibt bei diesem Verfahren in den überbrühten Pflanzenteilen. Die Anwendung organischer Lösungsmittel als Extraktionsmittel hat zahlreiche Nachteile, darunter besonders den, daß sich Reste des Lösungsmittels nur sehr schwer quantitativ vom Extrakt abtrennen lassen. Es hat daher nicht an Versuchen gefehlt, die Inhaltsstoffe der Kamille durch schonendere Extraktionsverfahren zu gewinnen. Durch die DE-OS 2 709 033 ist ein Verfahren bekannt, Kamille mit gesundheitlich unbedenklichen Gasen, z. B. auch mit $CO_2$ zu extrahieren, wobei die Gase sich in überkritischem Zustand befinden. Die Extraktion mit überkritischen Gasen erfordert jedoch die Anwendung von Temperaturen oberhalb der kritischen Temperatur des Extraktionsmittels. Bei der Anwendung von $CO_2$ bedeutet dies Temperaturen oberhalb 31,06°C zur Gewinnung des Gesamtextraktes. Der Extraktion mit überkritischem $CO_2$ liegt der Gedanke zugrunde, daß im überkritischen Bereich die Dichte des Extraktionsmittels und damit sein Lösevermögen durch die Anwendung hoher Extraktionsdrucke stark erhöht werden kann. Es hat sich jedoch gezeigt, daß wegen der extremen Temperaturempfindlichkeit einiger Kamille-Inhaltsstoffe, namentlich der Proazulene, z. B. des Matricins, dieses Extraktionsverfahren nicht voll befriedigt.

Es ist ferner bereits bekannt, Extraktionen mit flüssigem $CO_2$, also im Bereich unterhalb der kritischen Temperatur, durchzuführen. Eine Übersicht über den technischen Stand geben z. B., W. G. Schultz u. a. in »Food Technology, Vol. 24, 1282 ff (1970) und Vol. 28, 32 ff (1974). Wie sich aus der Literatur ergibt, wurde die Extraktion mit flüssigem $CO_2$ vor allem bei Kaffee, Gewürz- und Fruchtaromen angewendet. Auch für Hopfen wurde, z. B. in DE-AS 2 827 002 die Extraktion mit flüssigem $CO_2$ vorgeschlagen. Für die Extraktion der Kamille ist hingegen bisher nur das oben erwähnte Verfahren mit überkritischem $CO_2$ bekannt. Die Anwendung von flüssigem $CO_2$ ist bei Kamille auch nicht naheliegend, da in der Literatur stets die Selektivität des Lösevermögens von flüssigem $CO_2$, insbesondere für Verbindungen vom Typ der Ester, Aldehyde und Ketone sowie für Moleküle bis zu einem Molekulargewicht von ca. 150 hervorgehoben wird. Die Inhaltsstoffe der Kamille, z. B. das Matricin, das Bisabolol und die Flavonoide, weisen jedoch überwiegend höhere Molekulargewichte sowie freie Hydroxylgruppen auf.

Es bestand daher die Aufgabe, Kamille unter schonenden Bedingungen, insbesondere bei niedriger Temperatur und in kurzer Extraktionszeit so zu extrahieren, daß eine möglichst quantitative Ausbeute des naturbelassenen Gesamtextraktes erhalten wird.

Diese Aufgabe wurde erfindungsgemäß dadurch gelöst daß die Extraktion mit flüssigem Kohlendioxid bei Temperaturen unterhalb der kritischen Temperatur des Kohlendioxids und bei einem Druck durchgeführt wird, der bis zu 400 bar über dem Sättigungsdruck des Kohlendioxids der Extraktionstemperatur liegt. Die Abscheidung der Extrakte erfolgt durch Druck- und/oder Temperaturänderung in einem Expansionsgefäß, wobei eine Temperatur bei oder unterhalb der Extraktionstemperatur und als Druck der Sättigungsdruck des Kohlendioxids bei der Abscheidetemperatur gewählt werden. Auf diese Weise bildet sich im Entspannungsgefäß ein 2-Phasen-System, bestehend aus dem gasförmigen, extraktfreien Kohlendioxid und flüssigem, mit Extrakt beladenem Kohlendioxid, aus welchem das gasförmige extraktfreie Kohlendioxid kontinuierlich über Kompressoren in den Extraktionsbehälter zurückgeführt wird.

In überraschender Weise wird besonders durch die Anwendung von Extraktionsdrücken weit oberhalb des Sättigungsdruckes des Kohlendioxids eine quantitative Extraktion in kurzen Extraktionszeiten erzielt. Dies war nicht naheliegend, da im flüssigen Kohlendioxid durch Druckerhöhung nur eine unwesentliche Erhöhung der Dichte erreichbar ist und daher keine wesentliche Steigerung des Lösevermögens zu erwarten war. Tatsächlich wird in den zitierten Arbeiten von W. G. Schultz u. a. stets beim Sättigungsdruck des Kohlendioxids extrahiert.

Durch Anwendung der erfindungsgemäßen Verfahrensweise ergeben sich mannigfache Vorteile. So werden vor allem auch die thermolabilen Kamille-Inhaltsstoffe schonend und in hoher Ausbeute gewonnen. Die Extraktionszeit zur Gewinnung eines qualitativ hochwertigen Kamilleextraktes kann auf 3—5 Stunden begrenzt werden. Die Anwendung von Kosolventien, z. B. eine zusätzliche Eindosierung von Wasser, ist nicht erforderlich, trotzdem werden sowohl Duftstoffe als auch Aromasubstanzen und heilwirksame Inhaltsstoffe gleichzeitig und vollständig isoliert und ein Wachstum von unerwünschten Keimen und Schimmelpilzen wird auf diese Weise vermieden.

Die nachfolgenden Beispiele sollen das erfindungsgemäße Verfahren näher erläutern, ohne es jedoch hierauf zu beschränken.

Beispiele

Beispiel 1

a) Substrat:
Kamillenblüten, deutsch (Chamomillae flos)

| | | |
|---|---|---|
| Extraktgehalt (n-Hexan) | 4,1% | (des wasserfreien Substrats) |
| Ätherisches Öl | 0,7 ml/100 g | (des wasser- freien Sub- strats) |
| α-Bisabolol | 2,1 ppm | (des wasserfreien Substrats) |

b) Extraktionsbedingungen:

| | |
|---|---|
| Extraktionstemperatur | 29°C |
| Extraktionsdruck | 130 bar |
| Extraktionszeit | 3 Stunden |

c) Abscheidebedingungen:

| | |
|---|---|
| Temperatur | 22°C |
| Druck | ca. 60 at (~60 bar) |

d) Ausbeute

| | | |
|---|---|---|
| Gesamtextrakt | 1,4% | (des wasserfreien Substrats) |
| Ätherisches Öl | 0,5 ml/100 g | (des wasser- freien Sub- strats |
| α-Bisabolol | 1,3 ppm | (des wasserfreien Substrats) |

Beispiel 2

a) Substrat:
Kamillenblüten, deutsch (Chamomillae flos)
Zusammensetzung wie in Beispiel 1

b) Extraktionsbedingungen:

| | |
|---|---|
| Extraktionstemperatur | 28°C |
| Extraktionsdruck | 260 bar |
| Extraktionszeit | 5 Stunden |

c) Abscheidebedingungen
(wie in Beispiel 1)

d) Ausbeute:

| | | |
|---|---|---|
| Gesamtextrakt | 2,2% | (des wasserfreien Substrats) |
| Ätherisches Öl | 0,7 ml/100 g | (des wasser- freien Sub- strats) |
| α-Bisabolol | 1,9 ppm | (des wasserfreien Substrats) |

**Patentanspruch**

Verfahren zur Gewinnung von Inhaltsstoffen der Kamille durch Extraktion mit verflüssigtem Kohlendioxid, dadurch gekennzeichnet, daß die Extraktion bei einer Temperatur unterhalb der kritischen Temperatur des Kohlendioxids und bei einem Druck durchgeführt wird, der um bis zu 400 bar über dem Sättigungsdruck des Kohlendioxids bei der Extraktionstemperatur liegt und die Extrakte durch Druck- und/oder Temperaturänderung in einem Expansionsgefäß abgeschieden werden, wobei eine Temperatur bei oder unterhalb der Extraktionstemperatur und als Druck der Sättigungsdruck des Kohlendioxids bei der Abscheidetemperatur gewählt werden.

**Claim**

A process for recovering ingredients of camomile by extraction with liquefied carbon dioxide, characterized in that extraction is carried out at a temperature below the critical temperature of the carbon dioxide and unter a pressure up to 400 bars above the saturation pressure of the carbon dioxide at the extraction temperature and the extracts are separated by changing the pressure and/or temperature in an expansion vessel, the temperature selected being a temperature equal to or below the extraction temperature and the pressure selected being the saturation pressure of the carbon dioxide at the separation temperature.

**Revendication**

Procédé de récupération des substances contenues de la camomille par extraction avec du dioxyde de carbone liquéfié, caractérisé en ce qu'on exécute l'extraction à une température inférieure à la température critique du dioxyde de carbone et sous une pression qui se situe jusqu'à 400 bars au-dessus de la pression saturante du dioxyde de carbone à la température d'extraction, et en ce qu'on sépare les extraits par un changement de pression et/ou de température dans un récipient d'expansion, en choisissant une température égale ou inférieure à la température d'extraction et comme pression la pression saturante du dioxyde de carbone à la température de séparation.